(19) Europäisches Patentamt
European Patent Office
Office européen des brevets

(11) EP 0 994 868 B1

(12) **EUROPÄISCHE PATENTSCHRIFT**

(45) Veröffentlichungstag und Bekanntmachung des Hinweises auf die Patenterteilung:
**09.06.2004 Patentblatt 2004/24**

(21) Anmeldenummer: **98939610.6**

(22) Anmeldetag: **03.07.1998**

(51) Int Cl.7: **C07D 319/06**, C11B 9/00

(86) Internationale Anmeldenummer:
**PCT/EP1998/004107**

(87) Internationale Veröffentlichungsnummer:
**WO 1999/002515 (21.01.1999 Gazette 1999/03)**

(54) **VERFAHREN ZUR HERSTELLUNG VON OPTISCH AKTIVEM 5-METHYL-2-(1-METHYL-BUTYL)-5-PROPYL-1,3-DIOXAN**

METHOD FOR PREPARING OPTICALLY ACTIVE 5-METHYL-2-(1-METHYL-BUTYL)-5-PROPYL-1,3-DIOXAN

PROCEDE POUR LA PREPARATION DE 5-METHYL-2-(1-METHYL-BUTYL)-5-PROPYL-1,3-DIOXANNE A ACTION OPTIQUE

(84) Benannte Vertragsstaaten:
**CH DE ES FR GB IT LI NL**

(30) Priorität: **11.07.1997 DE 19729840**

(43) Veröffentlichungstag der Anmeldung:
**26.04.2000 Patentblatt 2000/17**

(73) Patentinhaber: **Kao Corporation**
**Chuo-Ku Tokyo 103-8210 (JP)**

(72) Erfinder:
- **MARKERT, Thomas**
**D-40789 Monheim (DE)**
- **MERKEL, Dirk**
**D-42697 Solingen (DE)**
- **ALTENBACH, Hans-Josef**
**D-42097 Wuppertal (DE)**

(74) Vertreter: **Wolf, Matthias**
**Hoffmann - Eitle**
**Patent- und Rechtsanwälte**
**Postfach 81 04 20**
**81904 München (DE)**

(56) Entgegenhaltungen:
**DE-A- 3 016 007**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

### Gebiet der Erfindung

Die Erfindung betrifft ein Verfahren zur Herstellung von optisch aktivem 5-Methyl-2-(1-methyl-butyl)-5-propyl-1,3-dioxan.

### Stand der Technik

Viele natürliche Riechstoffe stehen, gemessen am Bedarf, in völlig unzureichender Menge zur Verfügung. Beispielsweise sind zur Gewinnung von 1 kg Rosenöl 5.000 kg Rosenblüten notwendig; die Folgen sind eine sehr stark limitierte Weltjahresproduktion sowie ein hoher Preis. Es ist daher klar, daß die Riechstoffindustrie einen ständigen Bedarf an neuen Riechstoffen mit interessanten Duftnoten hat, um die Palette der natürlich verfügbaren Riechstoffe zu ergänzen und die notwendigen Anpassungen an wechselnde modische Geschmacksrichtungen vornehmen sowie den ständig steigenden Bedarf an Geruchsverbesserern für Produkte des täglichen Bedarfs wie Kosmetika und Reinigungsmittel decken zu können.

Darüber hinaus besteht generell ein ständiger Bedarf an synthetischen Riechstoffen, die sich günstig und mit gleichbleibender Qualität herstellen lassen und erwünschte olfaktorische Eigenschaften haben, d.h. angenehme, möglichst naturnahe und qualitativ neuartige Geruchsprofile von ausreichender Intensität besitzen und in der Lage sind, den Duft von kosmetischen und Verbrauchsgütern vorteilhaft zu beeinflussen. Mit anderen Worten: Es besteht ein ständiger Bedarf an Verbindungen, die charakteristische neue Geruchsprofile bei gleichzeitig hoher Haftfestigkeit, Geruchsintensität und Strahlkraft aufweisen.

5-Methyl-2-(1-methyl-butyl)-5-propyl-1,3-dioxan **22** (Handelsname Troënan®) ist ein bekannter

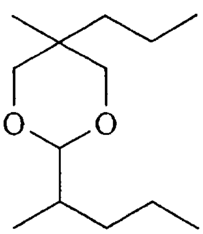

**22**

synthetischer Riechstoff, der ausgehend von 2-Methylpentanal **23**, zugänglich ist (vergleiche nachstehendes Schema). Dieses Edukt **23** wird durch gekreuzte Cannizzaro-Reaktion mit 2 Äquivalenten Formaldehyd zu 2-Methyl-2-propyl-1,3-propandiol **24** umgesetzt. Das Diol **24** kann nun unter Acetalisierung wieder mit 2-Methylpentanal **23** unter Bildung von Troënan® **22** reagieren.

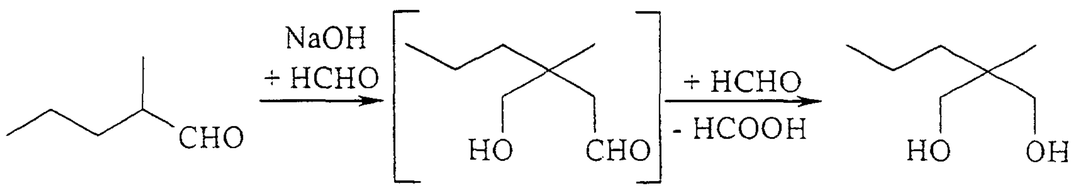


23 24

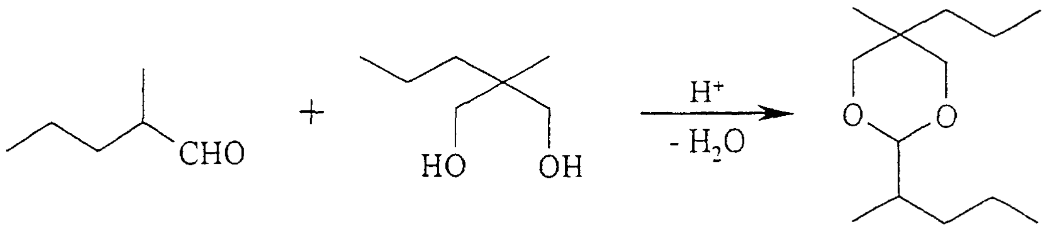


23 24 22

Troënan® wird aufgrund seiner Ligusterblüten-Note für blumige und würzige Formulierungen, insbesondere Maiglöckchen-Kompositionen geschätzt. Es hat den Vorteil gegenüber anderen Maiglöckchen-Komponenten, alkalistabil zu sein.

Troënan® **22** enthält zwei *cis*/*trans*-Isomere, die wiederum aus je zwei Enantiomeren bestehen:

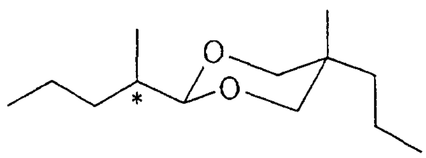

***trans*-22**

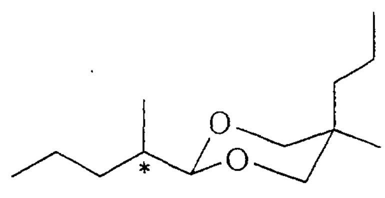

***cis*-22**

DE-A-3016007 beschreibt die Herstellung von racemischem 5-Methyl-2-(1-methyl-butyl)-5-propyl-1,3-dioxan als Riechstoff durch Umsetzung von 2-Methylpentanal, 1,3-Alkandiol, Orthoameisensäuretriethylester und Para-Toluolsulfonsäure und anschließendes Abdestillieren eines Gemisches aus Ameisensäureethylester und Ethanol.

**Beschreibung der Erfindung**

In orientierenden Untersuchungen der Anmelderin mittels "GC-Sniffing" hatte sich überraschend gezeigt, daß das *trans*-Isomere des Troënans® die typische Liguster-Note aufweist, während das *cis*-Isomere eine schwache blumig-bergamottene Note hat.

Aufgabe der vorliegenden Erfindung war es, ein Verfahren zur Herstellung von optisch aktivem 5-Methyl-2-(1-methyl-butyl)-5-propyl-1,3-dioxan bereitzusteller

Die genannte Aufgabe wurde durch ein Verfahren gemäß Patentanspruch gelöst.

Die erfindungsgemäße Synthese geht von kommerziell erhältlichem, enantiomerenreinem (*S*)-(-)-Milchsäuremethylester **56** aus, welcher im ersten Schritt zu einem *N,N*-disubstituierten Amid umgesetzt wird. Nach Schützen der OH-Funktion wird die Amid-Gruppe über eine Grignard-Reaktion in ein α,β-ungesättigtes Keton **56** umgewandelt. Die Hydroxygruppe wird nach dem Entfernen der Schutzgruppe und anschließender Acetalisierung in das Mesylat **57** umgewandelt. Nach der reduktiven 1,2-Umlagerung unter Erhalt der Stereochemie wird ein "Dehydro-Troënan" **58** über eine katalytische Hydrierung in das optisch aktive 5-Methyl-2-(1-methyl-butyl)-5-propyl-1,3-dioxan (-)-**22** überführt.

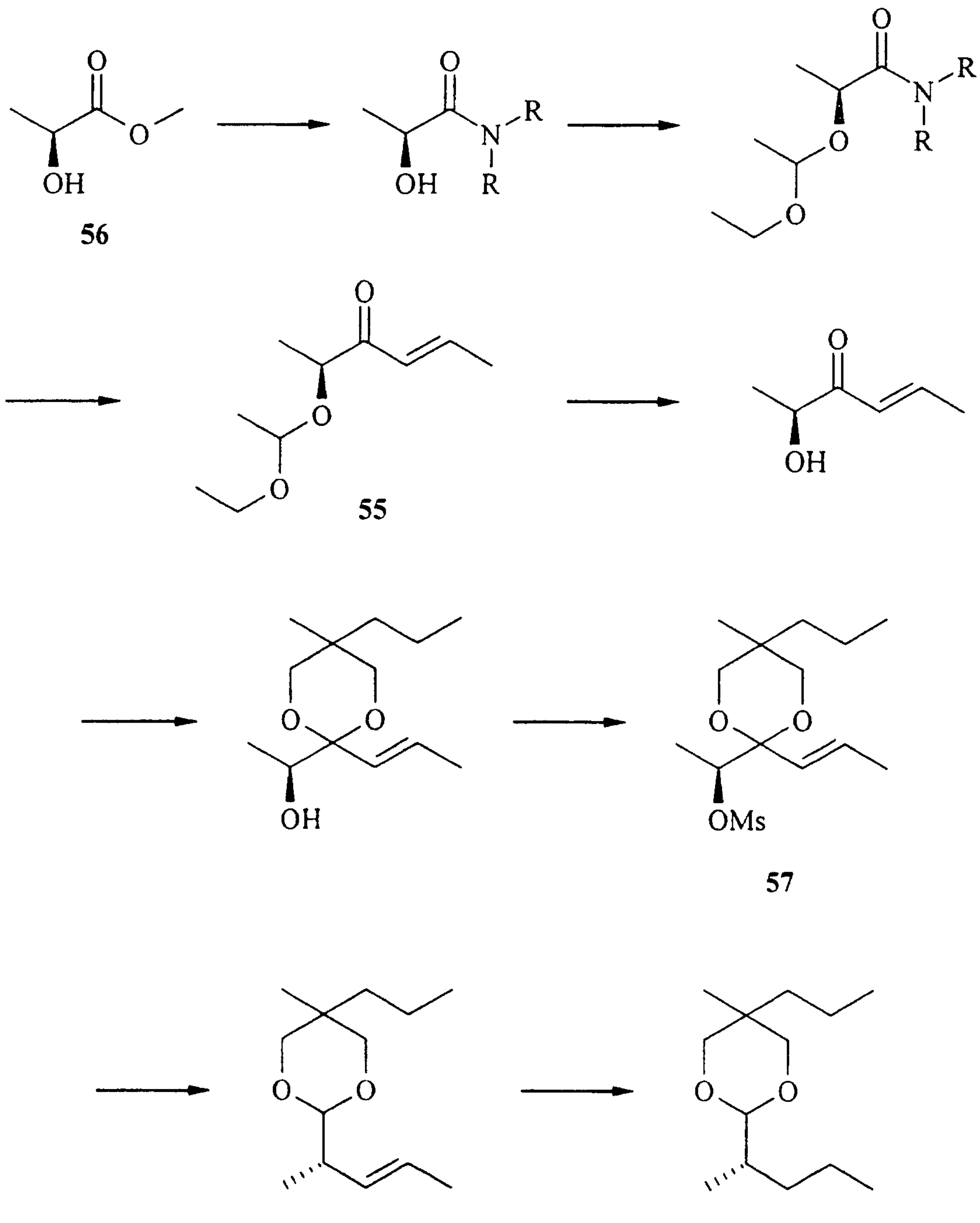

56

55

57

58

(-) - 22

Das $^{1}H$-NMR-Spektrum von (-)-**22** stimmt mit dem des *cis*/*trans*-Gemisches von Troënan® **22** überein.

Die Drehwertbestimmung ergab einen spezifischen Drehwert von $[\alpha]_D^{20}$ = -2.43 °.

Durch die Chiral-Pool-Synthese wurde ein optisch aktives (-)-**22** als *cis*/*trans*-Gemisch erhalten. Es zeigte sich, daß dieses (-)-**22** hinsichtlich seiner geruchlichen Eigenschaften überraschenderweise sowohl in qualitativer Hinsicht (typisches Geruchsprofil) als auch im Hinblick auf die Geruchsintensität (quantitativer Aspekt) handelsüblichem Troënan® überlegen war.

Gegenstand der vorliegenden Erfindung ist dementsprechend ein Verfahren zur Herstellung von optisch aktivem 5-Methyl-2-(1-methyl-butyl)-5-propyl-1,3-dioxan, indem man

- Milchsäuremethylester in das entsprechende *N*,*N*-disubstituierte Amid (Aminolyse mit Piperidin und/oder Pyrrolidin) überführt,
- dessen freie OH-Funktion durch Umsetzung mit Ethylvinylether in Gegenwart von p-Pyridiniumtosylat als Katalysator schützt,

- das so erhaltene N-Pentamethylen- bzw. N-Tetramethylenlactamid in üblicher Weise einer Grignard-Reaktion zum entsprechenden α,β-ungesättigtes Keton unterwirft,
- die in der zweiten Stufe eingeführte Schutzgruppe in üblicher Weise (beispielsweise durch Rühren mit Essigsäure) wieder abspaltet,
- die Ketonfunktion in üblicher Weise (zum Beispiel durch Umsetzung mit 2-Methyl-2-propyl-1,3-propandiol) acetalisiert,
- die freie OH-Funktion durch Umsetzung mit Methansulfonsäurechlorid in Pyridin mesyliert,
- das so erhaltene Zwischenprodukt in Gegenwart von Diisobutylaluminiumhydrid und Triethylaluminium einer reduktiven 1,2-Umlagerung unter Erhalt der Stereochemie unterwirft und
- die so erhaltene Dehydro-Verbindung durch katalytische Hydrierung in das optisch aktive 5-Methyl-2-(1-methylbutyl)-5-propyl-1,3-dioxan überführt.

Das nach dem Verfahren der vorliegenden Erfindung erhältliche optisch aktive 5-Methyl-2-(1-methyl-butyl)-5-propyl-1,3-dioxan zeigt eine überraschend hohe Strahlkraft und besticht durch seine intensive Liguster und Muguet-Note.

Die nachfolgenden Beispiele dienen der Erläuterung der Erfindung und sind nicht einschränkend zu verstehen.

## Beispiele

### 1.1 Allgemeine Angaben

Es wurden folgende Geräte und Materialien eingesetzt:

Schmelzpunkte:

Die angegebenen Schmelzpunkte wurden mit einem Schmelzpunkt-Mikroskop (Thermovar der Firma Reichert) bestimmt und sind nicht korrigiert.

Drehwerte:

Die Drehwerte wurden mit einem Polarimeter 241 der Firma Perkin-Elmer gemessen. Die in Verbindung mit dem spezifischen Drehwert angegebene Konzentration c ist als Substanzmenge (in g) pro 100 ml Lösung definiert.

IR-Spektroskopie:

Die IR-Spektren wurden mit einem Spektrometer 983 der Firma Perkin-Elmer aufgenommen.
Die Intensitäten der angegebenen Absorptionsbanden werden durch die Symbole s (stark), m (mittel) und w (wenig intensiv) beschrieben.

NMR-Spektroskopie

Die NMR-Spektren wurden mit den Spektrometern ARX 400, AMX 400 und AC 200 F der Firma Bruker aufgenommen. Die chemischen Verschiebungen sind in δ-Werten bezogen auf den Standard angegeben. Bei dem Gerät ARX 400 wurde das verwendete Lösungsmittel als Standard verwendet; bei den Geräten AMX 400 und AC 200 F wurde TMS als interner Standard verwendet.

Die Signalmultiplizitäten werden durch die Symbole s (Singulett), d (Dublett), t (Triplett), ψ-t (Pseudotriplett), q (Quartett) und m (Multiplett) charakterisiert. Um eine eindeutige Zuordnung der Wasserstoff- und Kohlenstoffatome zu ermitteln, wurden
2-D-COSY-Spektren ($^1H/^1H$- und $^1H/^{13}C$-), sowie DEPT-Spektren aufgenommen.
Die Numerierung der einzelnen Atome erfolgte möglichst einheitlich, um strukturelle Ähnlichkeiten und Variationen auch im Spektrum anschaulich zu machen; sie entspricht daher nicht immer IUPAC-Regeln.
Bei Verbindungen, die als *cis*/*trans*-Gemische vorliegen, wurden die Signale mit c für *cis* und t für *trans* gekennzeichnet, sofern eine Zuordnung möglich war.

Massenspektroskopie:

Die Aufnahme der Massenspektren erfolgte mit dem Gerät Varian MAT 311 A.

Elementaranalyse:

Die Elementaranalyse erfolgte mit dem Mikroelementar Analysator 240B der Firma Perkin-Elmer.

Chromatographische Verfahren:

Die gaschromatographische Reaktionskontrolle wurde mit einem Hewlett-Packard GC 5710 A an einer Kapillarsäule DB-5 (l = 30 m) durchgeführt.
Die Untersuchungen an einer chiralen GC-Säule wurde an einem Shimadzu GC-14A an einer Kapillarsäule Cyclodex-beta-I/P (l = 25 m) durchgeführt.
Die Messungen an einer chiralen HPLC-Säule erfolgten mit einem Gerät der Firma Merck L7100/L7400 an einer OD-R-Säule (l = 250 mm, d = 4 mm). Als Laufmittel wurde ein Gemisch von Acetonitril : Wasser = 50 : 50 bei einem Flow von 0.8 ml / min verwendet. Die Detektion erfolgte mittels eines UV-Detektors bei 254 nm.

### 1.2 Allgemeine Arbeitsvorschriften

Bei den unter 1.3 beschriebenen Synthesen kamen unter anderem folgende allgemeine Arbeitsvorschriften zum Einsatz. Sie wurden jeweils abkürzend mit AAV1 bis AAV3 bezeichnet. Diese Vorschriften sind wie folgt:

AAV 1: Acetalisierung

1 Äquivalent des Aldehyds und 1 Äquivalent des Diols werden mit 0.025 Äquivalenten *p*-Toluolsulfonsäure-Hydrat in Cyclohexan gelöst und in einer Dean-Stark-Apparatur (Wasserabscheider) unter Rückfluß erhitzt, wobei sich das entstandene Wasser abscheidet. Nach Beendigung der Reaktion wird die erkaltete Lösung mit gesättigter Natriumhydrogencarbonat-Lösung ausgeschüttelt, bis das Waschwasser nicht mehr alkalisch ist. Die Lösung wird über Natriumsulfat getrocknet und das Lösungsmittel am Rotationsverdampfer bei vermindertem Druck abgezogen. Der Rückstand wird im Ölpumpenvakuum fraktioniert.

AAV 2: Mesylierung

1 Äquivalent des Alkohols wird in *n* ml Pyridin gelöst und auf 0 °C gekühlt. Es werden 1.1 Äquivalente Methansulfonsäurechlorid zugetropft und die Mischung 24 h bei 4 °C im Kühlschrank belassen. Man gießt auf 2*n* ml Eis und neutralisiert mit halbkonz. Salzsäure. Die Lösung wird mit Ethylacetat extrahiert. Nach Waschen mit ges. Natriumhydrogencarbonat-Lösung und Trocknen über Natriumsulfat wird das Lösungsmittel am Rotationsverdampfer abgezogen.

AAV 3: Aminolyse

1 Äquivalent (*S*)-(-)-Methyllactat **56** und 2 Äquivalente des Amins werden in einer Destillationsapparatur bis auf 105 °C erhitzt. Das entstehende Methanol wird über eine 15 cm Vigreux-Kolonne abdestilliert. Nach Beendigung der Reaktion wird das überschüssige Amin abdestilliert und das Produkt in der gleichen Apparatur bei 0.1 mbar fraktioniert.

### 1.3 Chiral-Pool-Synthese

#### 1.3.1 Synthese von *N*-Pentamethylen-lactamid 59

Nach AAV 3 werden 47.6 g (*S*)-(-)-Methyllactat **56** (0.46 mol) mit 78.2 g Piperidin (0.92 mol) umgesetzt. Nach Destillation erhält man eine klare, farblose Flüssigkeit.
Ausbeute: 73 %
Sdp.: 111 °C / 0.8 mbar
Drehwert: $[\alpha]_D^{20}$ = - 1.13 ° (c = 12.7)

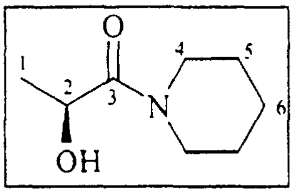

**$^1$H-NMR** ($CDCl_3$, 200 MHz): δ = 4.4 (q, 1H; H-2), 3.6/3.35 (t. 4H, H-4 diastereotop), 1.6 (m, 6H, H-5/H-6), 1.3 (d, 3H, H-1)

**$^{13}$C-NMR** ($CDCl_3$, 50 MHz): δ = 173.9 (C-3), 64.0 (C-2), 45.8/43.5 (C-4). 26.2/25.5 (C-5), 24.4 (C-6), 21.4 (C-1)

**MS** (70 eV): m/z (%) = 157 (18) [$M^+$], 114 (12) [$C_5H_8O_2N^+$], 113 (65) [$C_6H_{11}ON^+$], 112 (100) [$C_6H_{10}ON^+$], 85 (13) [$C_5H_{11}N^+$], 84 (15) [$C_5H_{10}N^+$], 69 (83) [$C_5H_9^+$], 56 (13) [$C_3H_6N^+$], 55 (12) [$C_3H_3O^+$], 45 (33) [$C_2H_5O^+$], 42 (18) [$C_2H_4N^+$], 41 (63) [$C_3H_5^+$]

**IR (Film):**

| $\tilde{\nu}$ [$cm^{-1}$] | Schwingungstyp |
|---|---|
| 3415, s | O-H Valenzschwingung |
| 2940, s | C-H Valenzschwingung |
| 1635, s | C=O Valenzschwingung, Amid |
| 1480, s + 1400, s | C-H Deformationsschwingung |
| 1115, s | C-O Valenzschwingung |

**EA:**

| | |
|---|---|
| $C_8H_{15}NO_2$ | M = 157.21 g/mol |
| berechnet: | C = 61.12 % H = 9.62 % N = 8.91 % |
| gefunden: | C = 60.31 % H = 9.63 % N = 8.79 % |

**1.3.2 Synthese von *N*-Tetramethylen-lactamid 60**

Nach AAV 3 werden 31.2 g (*S*)-(-)-Methyllactat **56** (0.3 mol) mit 42.7 g Pyrrolidin (0.6 mol) umgesetzt. Nach Destillation erhält man eine klare, farblose Flüssigkeit.
Ausbeute: 97 %
Sdp.: 95 °C / 0.1 mbar
Drehwert: $[\alpha]_D^{20}$ = - 48.67 ° (c = 12.8)

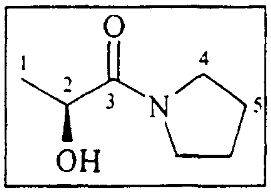

**$^1$H-NMR** ($CDCl_3$, 200 MHz): δ = 4.35 (q, 1H; H-2), 3.65/3.3 (m, 4H, H-4 diastereotop), 2.1-1.8 (m, 4H, H-5), 1.35 (d, 3H, H-1)

**$^{13}$C-NMR** ($CDCl_3$, 50 MHz): δ = 173.5 (C-3), 65.6 (C-2), 46.2/46.0 (C-4), 26.1/23.9 (C-5), 20.6 (C-1)

**MS** (70 eV): m/z (%) = 157 (18) [$M^+$], 114 (12) [$C_5H_8O_2N^+$], 113 (65) [$C_6H_{11}ON^+$], 112 (100) [$C_6H_{10}ON^+$], 85 (13) [$C_5H_{11}N^+$], 84 (15) [$C_5H_{10}N^+$], 69 (83) [$C_5H_9^+$], 56 (13) [$C_3H_6N^+$], 55 (12) [$C_3H_3O^+$], 45 (33) [$C_2H_5O^+$], 42 (18) [$C_2H_4N^+$], 41 (63) [$C_3H_5^+$]

**IR (Film):**

| $\tilde{\nu}$ [$cm^{-1}$] | Schwingungstyp |
|---|---|
| 3405, s | O-H Valenzschwingung |
| 2975, s | C-H Valenzschwingung |
| 1635, s | C=O Valenzschwingung, Amid |
| 1435, s + 1380, s | C-H Deformationsschwingung |
| 1128, s | C-O Valenzschwingung |

**EA:**

| | | |
|---|---|---|
| $C_7H_{13}NO_2$ | M = 143.19 g/mol | |
| berechnet: | C = 58.72 % H = 9.15 % N = 9.78 % | |
| gefunden: | C = 58.29 % H = 9.23 % N = 9.58 % | |

**1.3.3 Synthese des EE-geschützten *N*-Pentamethylen-lactamids 62**

In 200 ml Dichlormethan$_{abs.}$ werden 46.0 g *N*-Pentamethylen-lactamid **59** (293 mmol). 76.6 g Ethylvinylether und 7.53 g *p*-Pyridiniumtosylat (30 mmol) gelöst. Die Lösung wird 3 h am Rückfluß erhitzt. Nach dem Abkühlen wird mit dest. Wasser und ges. Natriumhydrogencarbonat-Lösung gewaschen. Das Lösungsmittel wird einrotiert und der Rückstand über eine 15 cm Vigreux-Kolonne fraktioniert. Man erhält eine klare, farblose Flüssigkeit.
Ausbeute: 45 %
Sdp.: 78 °C / 0.08 mbar
Drehwert: $[\alpha]_D^{20}$ = - 47.77 ° (c = 17.6)

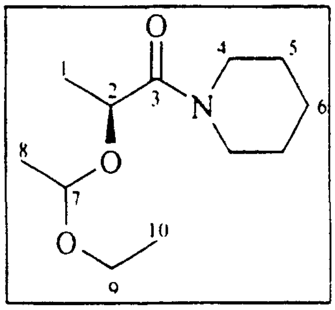

**$^1$H-NMR** ($CDCl_3$, 200 MHz): δ = 4.80/4.70 (q, 1H; H-2), 4.6/4.5 (q, 1H, H-7), 3.55 (m, 6H, H-4/H-9), 1.65 (m, 6H, H-5/H-6),
1.45-1.30 (m, 6H, H-1/H-8), 1.2 (m, 3H, H-10) Die Protonen an $C^2$ und $C^7$ spalten aufgrund des vorliegenden Diastereomerengemischs auf.
**$^{13}$C-NMR** ($CDCl_3$, 50 MHz): δ = 170.8 (C-3), 99.3/98.9 (C-7), 70.4 (C-2), 61.3/60.3 (C-9), 46.1/43.2 (C-4), 26.4/25.7 (C-5), 24.6 (C-6), 20.2 (C-8), 18.8/18.4 (C-1), 15.3 (C-10)
**IR (Film):**

| $\tilde{\nu}$ [$cm^{-1}$] | Schwingungstyp |
|---|---|
| 2935, s | C-H Valenzschwingung |
| 1640, s | C=O Valenzschwingung, Amid |
| 1440, s + 1375, m | C-H Deformationsschwingung |
| 1080, s | C-O Valenzschwingung |

**1.3.4 Synthese des EE-geschützten *N*-Tetramethylen-lactamids 61**

In 150 ml Dichlormethan$_{abs.}$ werden 34.7 g *N*-Tetramethylen-lactamid **60** (243 mmol), 36.0 g Ethylvinylether (0.5 mol) und 2.3 g *p*-Pyridiniumtosylat (9 mmol) gelöst. Die Lösung wird 3 h bei Raumtemperatur gerührt und anschließend mit dest. Wasser und ges. Natriumhydrogencarbonat-Lösung gewaschen. Das Lösungsmittel wird einrotiert und der Rückstand über eine 15 cm Vigreux-Kolonne fraktioniert. Man erhält eine klare, farblose Flüssigkeit.
Ausbeute: 76 %

Sdp.: 105 °C / 0.1 mbar
Drehwert: $[\alpha]_D^{20}$ = - 76.84 ° (c = 10.2)

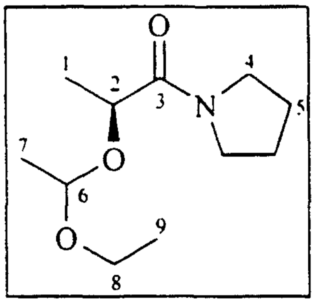

**$^1$H-NMR** ($CDCl_3$, 200 MHz): δ = 4.80/4.75 (q, 1H; H-2), 4.5/4.35 (q, 1H, H-6), 3.7-3.3 (m, 6H, H-4/H-8), 2.05-1.75 (m, 4H, H-5), 1.4 (m, 6H, H-1/H-7), 1.15 (m, 3H, H-9)
Die Protonen an $C^2$ und $C^6$ spalten aufgrund des vorliegenden Diastereomerengemischs auf.
**$^{13}$C-NMR** ($CDCl_3$, 50 MHz): δ = 171.3 (C-3), 99.2/98.5 (C-6), 70.4/69.9 (C-2), 61.0/60.0 (C-8), 46.1 (C-4), 26.4/23.8 (C-5), 20.0 (C-7), 18.2/17.9 (C-1), 15.4/15.2 (C-9)
**IR (Film):**

| $\tilde{\nu}$ [$cm^{-1}$] | Schwingungstyp |
|---|---|
| 2980, s | C-H Valenzschwingung |
| 1655, s | C=O Valenzschwingung, Amid |
| 1430, s + 1370, m | C-H Deformationsschwingung |
| 1085, s | C-O Valenzschwingung |

**1.3.5 Synthese von 2-Hydroxy-hex-4-en-3-on 63**

In 25 ml THF werden 3.6 g Magnesium-Späne (150 mmol) vorgelegt und 18.2 g 1-Brom-1-propen (150 mmol) in 50 ml THF innerhalb 30 min zugetropft. Es werden 30 min. bei 35 °C nachgerührt und anschließend 10.75 g des EE-geschützten *N*-Tetramethylen-lactamids **61** (50 mmol)n 25 ml THF, zugetropft. Es werden 3 h bei 40 °C nachgerührt. Nach dem Abkühlen der Lösung wird mit 2n Salzsäure hydrolysiert. Die Phasen werden getrennt und die wäßrige Phase wird mit Diethylether extrahiert. Die vereinigten organischen Phasen werden mit ges. Natriumhydrogencarbonat-Lösung neutralisiert. Nach dem einrotieren des Lösungsmittels kann aus dem Rückstand ein Teil des EE-geschützten 2-Hydroxy-hex-4-en-3-on **55** isoliert werden. Es wird wie folgt charakterisiert.
Sdp.: 62 °C / 0.1 mbar

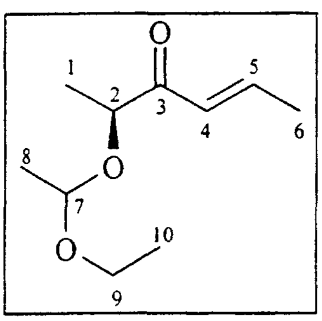

**$^1$H-NMR** ($CDCl_3$, 200 MHz): δ = 7.0 (dq, 1H; H-5), 6.5 (ψ-t, 1H, H-4), 4.75/4.65 (q, 1H, H-7), 4.35/4.2 (q, 1H, H-2), 3.65-3.35 (m, 2H, H-9), 1.95 (d, 3H, H-6), 1.35 (m, 6H, H-1/H-8), 1.15 (ψ-q, 3H, H-10)

Die Protonen an $C^2$ und $C^7$ spalten aufgrund des vorliegenden Diastereomerengemischs auf
**$^{13}$C-NMR** ($CDCl_3$, 50 MHz): $\delta$ = 200.5 (C-3), 144.5/143.5 (C-5), 126.6 (C-4), 99.8/99.5 (C-6), 76.1 (C-2), 61.7/60.7 (C-9). 20.1 (C-8), 18.4 (C-1), 16.1/15.3 (C-10)
**IR (Film):**

| $\tilde{\nu}$ [$cm^{-1}$] | Schwingungstyp |
|---|---|
| 2980, s | C-H Valenzschwingung, aliphat. |
| 1695, s | C=O Valenzschwingung, $\alpha,\beta$-unges. Keton |
| 1630, s | C=C Valenzschwingung, $\alpha,\beta$-unges, Keton |
| 1445, s + 1375, m | C-H Deformationsschwingung |
| 1080, s | C-O Valenzschwingung |

Die geschützte Verbindung **55** wird mit 40 ml 10 %iger Essigsäure 30 min am Rückfluß erhitzt. Nach dem Abkühlen wird mit Dichlormethan extrahiert. Nach Trocknen über Kaliumcarbonat wird das Lösungsmittel abgezogen und der Rückstand über eine 15 cm Vigreux-Kolonne fraktioniert. Man erhält eine klare, hellgelbe Flüssigkeit.
Ausbeute: 54 %
Sdp.: 98 °C / 40 mbar
Drehwert: $[\alpha]_D^{20}$ = + 40.25 ° (c = 3.5)

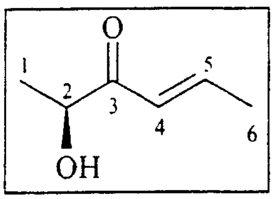

**$^{1}$H-NMR** ($CDCl_3$, 200 MHz): $\delta$ = 7.05 (dq, 1H; H-5), 6.25 (d, 1H, H-4), 4.45 (q, 1H, H-2), 1.95 (d, 3H, H-6), 1.40 (d, 3H, H-1)
**$^{13}$C-NMR** ($CDCl_3$, 50 MHz): $\delta$ = 201.1 (C-3), 145.5 (C-5), 126.4 (C-4), 71.3 (C-2), 20.5 (C-6), 18.2 (C-1)
**IR (Film):**

| $\tilde{\nu}$ [$cm^{-1}$] | Schwingungstyp |
|---|---|
| 3445, s | O-H Valenzschwingung |
| 3025, w | C-H Valenzschwingung, olefin. |
| 2980, s | C-H Valenzschwingung, aliphat. |
| 1690, s | C=O Valenzschwingung, $\alpha,\beta$-unges, Keton |
| 1630, s | C=C Valenzschwingung, $\alpha,\beta$- unges. Keton |
| 1440, s + 1375, s | C-H Deformationsschwingung |
| 1070, s | C-O Valenzschwingung |

**1.3.6 Synthese von 2-Hydroxyethyl-5-methyl-2-(1-propenyl)-5-propyl-1,3-dioxan 64**

Nach AAV 1 werden 2.9 g 2-Hydroxy-hex-4-en-3-on **63** (25 mmol) und 3.3 g 2-Methyl-2-propyl-1,3-propandiol **24** (25 mmol) in 50 ml Cyclohexan acetalisiert.
Nach Destillation über eine Drehband-Kolonne wird eine klares, hellgelbes Öl erhalten.
Ausbeute: 40 %

Sdp.: 73 °C / 0.1 mbar

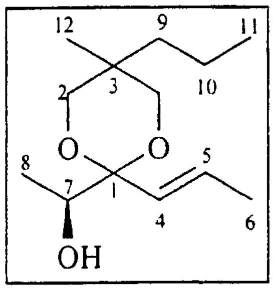

**$^1$H-NMR** ($CDCl_3$, 400.1 MHz): δ = 5.85 (dq, 1H; H-5), 5.25 (d, 1H, H-4), 3.7-3.3 (m, 5H, H-2/H-7), 1.8 (d, 3H, H-6), 1.3 (m, 2H, H-9), 1.20/0.60 (s, 3H, H-12), 1.15 (d, 3H, H-8), 1.0 (q, 2H, H-10), 0.9 (t, 3H, H-11)

**$^{13}$C-NMR** ($CDCl_3$, 100.1 MHz): δ = 132.5 (C-5), 127.2 (C-4), 100.5 (C-1), 73.3 (C-7), 70.9/69.9 (C-2), 38.9/36.7 (C-9), 32.7 (C-3), 20.1/19.4 (C-12), 17.8 (C-6), 16.8 (C-10), 16.1 (C-8), 14.7 (C-11)

**MS** (70 eV): m/z (%) = 228 (1) [$M^+$], 183 (65) [$M^+ - C_2H_5O^+$], 97 (22) [$C_6H_9O^+$], 69 (100) [$C_4H_5O^+$], 56 (14) [$C_4H_8^+$], 55 (68) [$C_3H_3O^+$], 45 (11) [$C_2H_5O^+$], 43 (33) [$C_3H_7^+$], 41 (27) [$C_3H_5^+$]

**IR (Film):**

| $\tilde{\nu}$ [$cm^{-1}$] | Schwingungstyp |
|---|---|
| 3495, s | O-H Valenzschwingung |
| 3020, w | C-H Valenzschwingung, olefin. |
| 2960, s | C-H Valenzschwingung, aliphat. |
| 1670, w | C=C Valenzschwingung |
| 1470, m + 1390, m | C-H Deformationsschwingung |
| 1120, s | C-O Valenzschwingung |

**1.3.7 Mesylierung von 2-Hydroxyethyl-5-methyl-2-(1-propenyl)-5-propyl-1,3-dioxan 64**

Nach AAV 2 werden 12.6 g 2-Hydroxyethyl-5-methyl-2-(1-propenyl)-5-propyl-1,3-dioxan **64** (55 mmol) mit 7.0 g Methansulfonsäurechlorid (61 mmol) in 80 ml Pyridin mesyliert. Man erhält ein klares, hellbraunes Öl.
Ausbeute: 92 %
Sdp.: Zersetzung bei T > 105 °C

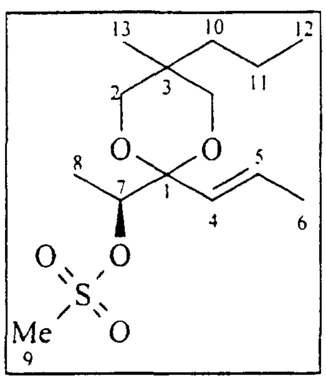

**$^1$H-NMR** ($CDCl_3$, 200 MHz): δ = 5.9 (dq, 1H; H-5), 5.2 (d, 1H, H-4), 4.55 (q, 1H, H-7), 3.7-3.3 (m, 4H, H-2), 3.05 (s, 3H, H-9), 1.85 (d, 3H, H-6), 1.6 (m, 2H, H-10), 1.35 (d, 3H, H-8), 1.15/0.65 (s, 3H, H-13), 1.0 (q, 2H, H-11), 0.9 (t, 3H, H-12)

**$^{13}$C-NMR** ($CDCl_3$, 50 MHz): δ = 133.9 (C-5), 125.9 (C-4), 98.8 (C-1), 83.1 (C-7), 69.8 (C-2), 28.7/36.6 (C-10), 38.4 (C-9). 32.9 (C-3), 19.6/19.0 (C-13), 17.8 (C-6), 16.7/15.7 (C-11), 15.9 (C-8), 14.9 (C-12)

**IR (Film):**

| $\tilde{\nu}$ [$cm^{-1}$] | Schwingungstyp |
|---|---|
| 3020, m | C-H Valenzschwingung, olefin. |
| 2960, s | C-H Valenzschwingung, aliphat. |
| 1670, w | C=C Valenzschwingung |
| 1440, s + 1360, s | C-H Deformationsschwingung |
| 1415, m + 1175, s | Sulfonat-Gruppe |
| 1110, s | C-O Valenzschwingung |

**1.3.8 Reduktive 1,2-Umlagerung**

In 40 ml Toluol$_{abs.}$ werden 4 g des Mesylats **57** (13 mmol) unter Stickstoff vorgelegt. Die Lösung wird auf -35 °C gekühlt und anschließend werden 30.5 ml DI-BAH-Lösung (1M in Hexan) und 22.5 ml Triethylaluminium-Lösung (1M in Hexan) über eine Spritze zudosiert. Die Lösung wird 2 h bei - 35 °C gerührt. Bei einer Temperatur von - 20 °C wird vorsichtig mit 15 ml ges. Natriumhydrogencarbonat-Lösung gequencht. Nach Zugabe von 10 ml dest. Wasser wird der Niederschlag abgesaugt und das Filtrat mit Diethylether extrahiert. Nach Trocknen über Natriumsulfat wird das Lösungsmittel einrotiert und der Rückstand im Kugelrohrofen destilliert. Man erhält eine klare, farblose Flüssigkeit
Ausbeute: 38 %
Sdp.: 90 - 100 °C / 0.1 mbar
Drehwert: $[\alpha]_D^{20}$ = + 6.28 ° (c = 6.2)

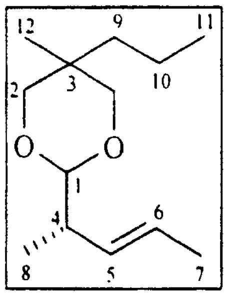

**$^1$H-NMR** ($CDCl_3$, 200 MHz): $\delta$ = 5.5 (m, 2H; H-5/H-6), 4.25 ($\psi$-t, 1H, H-1), 3.75 (d, 1H, H-2$c_{ax}$), 3.65 (d, 1H, H-2$t_{ax}$), 3.40 (d, 1H, H-2$t_{eq}$), 3.35 (d, 1H, H-2$c_{eq}$), 2.35 (m, 1H, H-4), 1.7 (d, 3H, H-7), 1.55 (m, 2H, H-9), 1.25 (m, 2H, H-10), 1.15 (s, 1.5H, H-12t), 1.05 (d, 3H, H-8), 0.9 (m, 3H, H-11), 0.65 (s, 1.5H, H-12c)

**$^{13}$C-NMR** ($CDCl_3$, 50 MHz): $\delta$ = 132.6 (C-6), 125.3 (C-5), 104.7 (C-1), 76.2/75.8 (C-2), 41.2 (C-4), 38.8/37.2 (C-10), 32.7 (C-3), 20.2/19.1 (C-12), 18.8 (C-7), 16.9/16.0 (C-10), 14.9 (C-11), 14.7 (C-8)

**MS** (70 eV): m/z (%) = 212 (2) [$M^+$], 143 (60) [$C_8H_{15}O_2^+$], 97 (39 ) [$C_6H_9O^+$], 69 (22) [$C_5H_9^+$], 56 (18) [$C_4H_8^+$], 55 (100) [$C_4H_7^+$], 43 (16) [$C_3H_7^+$], 41 (29) [$C_3H_5^+$]

**IR (Film):**

| $\tilde{\nu}$ [$cm^{-1}$] | Schwingungstyp |
|---|---|
| 3020, w | C-H Valenzschwingung, olefin. |
| 2960, s | C-H Valenzschwingung, aliphat. |
| 1465, m + 1385, m | C-H Deformationsschwingung |
| 1115, s | C-O Valenzschwingung |

**1.3.9 Synthese von optisch aktivem (-)-22**

In 80ml Cyclohexan werden 4.0 g 5-Methyl-2-(1-methyl-2-butenyl)-5-propyl-1,3-dioxan **58** (19 mmol) gelöst und nach Zugabe von 0.4 g Hydrierkatalysator (5 % Palladium auf Kohle) in einen Stahl-Autoklaven überführt. Die Mischung wird 7 h bei einem Druck von 10 bar Wasserstoff und einer Temperatur von 80 °C gerührt. Nach dem Abfiltrieren des Katalysators und Abziehen des Lösungsmittels wird der Rückstand im Kugelrohrofen destilliert. Es wird eine klare, farblose Flüssigkeit isoliert.
Ausbeute: 81 %
Sdp.: 75 - 90 °C / 0.1 mbar
Drehwert: $[\alpha]_D^{20}$ = - 2.43 ° (c = 5.2)

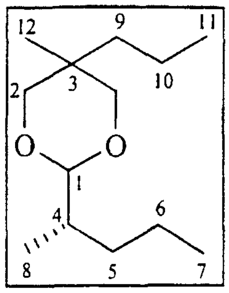

**$^1$H-NMR** ($CDCl_3$, 200 MHz): $\delta$ = 4.25 ($\psi$-t, 1H; H-1), 3.75 (d, 1H, H-$2c_{ax}$), 3.60 (d, 1H, H-$2t_{ax}$), 3.40 (d, 1H, H-$2t_{eq}$), 3.35 (d, 1H, H-$2c_{eq}$), 1.75-1.1 (m, 9H, H-4/H-5/H-6/H-9/H-10), 1.15 (s, 1.5H, H-12t), 1.05-0.8 (m, 9H, H-7/H-8/H-11), 0.65 (s, 1.5H, H-12c)

**$^{13}$C-NMR** ($CDCl_3$, 50 MHz): $\delta$ = 105.3 (C-1), 76.4/75.8 (C-2), 38.6/33.6 (C-9), 36.9 (C-4), 32.7 (C-3), 20.3 (C-6), 18.8/16.7 (C-12), 15.9 (C-10), 15.0 (C-11), 14.2 (C-7), 13.9 (C-8)

## Patentansprüche

**1.** Verfahren zur Herstellung von optisch aktivem 5-Methyl-2-(1-methyl-butyl)-5-propyl-1,3-dioxan, **dadurch gekennzeichnet, daß** man

- Milchsäuremethylester in das entsprechende *N,N*-disubstituierte Amid (Aminolyse mit Piperidin und/oder Pyrrolidin) überführt,
- dessen freie OH-Funktion durch Umsetzung mit Ethylvinylether in Gegenwart von p-Pyridiniumtosylat als Katalysator schützt,
- das so erhaltene N-Pentamethylen- bzw. N-Tetramethylenlactamid in üblicher Weise einer Grignard-Reaktion zum entsprechenden $\alpha,\beta$-ungesättigtes Keton unterwirft,
- die in der zweiten Stufe eingeführte Schutzgruppe in üblicher Weise (beispielsweise durch Rühren mit Essigsäure) wieder abspaltet,
- die Ketonfunktion in üblicher Weise (zum Beispiel durch Umsetzung mit 2-Methyl-2-propyl-1,3-propandiol) acetalisiert,
- die freie OH-Funktion durch Umsetzung mit Methansulfonsäurechlorid in Pyridin mesyliert,
- das so erhaltene Zwischenprodukt in Gegenwart von Diisobutylaluminiumhydrid und Triethylaluminium einer reduktiven 1,2-Umlagerung unter Erhalt der Stereochemie unterwirft und
- die so erhaltene Dehydro-Verbindung durch katalytische Hydrierung in das optisch aktive 5-Methyl-2-(1-methyl-butyl)-5-propyl-1,3-dioxan überführt.

## Claims

**1.** Process for producing optically active 5-methyl-2-(1-methyl-butyl)-5-propyl-1,3-dioxane, **characterised in that**

- methyl lactate is converted into the corresponding *N*,*N*-disubstituted amide (aminolysis with piperidine and/or pyrrolidine),
- its free OH function is protected by reaction with ethyl vinyl ether in the presence of p-pyridinium tosylate as catalyst,
- the N-pentamethylene lactamide or N-tetramethylene lactamide thus obtained is subjected in conventional manner to a Grignard reaction to form the corresponding $\alpha,\beta$-unsaturated ketone,
- the protective group introduced in the second stage is cleaved again in conventional manner (for example by stirring with acetic acid),
- the ketone function is acetalised in conventional manner (for example by reaction with 2-methyl-2-propyl-1,3-propandiol),
- the free OH function is mesylated by reaction with methane sulphochloride in pyridine,
- the intermediate product thus obtained is subjected to a reductive 1,2-rearrangement in the presence of diisobutyl aluminium hydride and triethyl aluminium while maintaining the stereochemistry and

- the dehydro compound thus obtained is converted by catalytic hydrogenation into the optically active 5-methyl-2-(1-methyl-butyl)-5-propyl-1,3-dioxane.

## Revendications

**1.** Procédé de production de 5-méthyl-2-(1-méthyl-butyl)-5-propyl-1,3-dioxane optiquement actif, **caractérisé en ce qu'**

- on transforme l'ester méthylique d'acide lactique en amide N,N-disubstitué correspondant (aminolyse avec la pipéridine et/ ou la pyrolidine),
- on protège sa fonction OH libre par réaction avec l'ester éthylvinylique en présence de p-tosylate de pyridinium comme catalyseur,
- on soumet le N-pentaméthylène ou le N-tetraméthylènelactamide d'une manière habituelle à une réaction de Grignard, en cétone $\alpha$, $\beta$ - non saturé correspondant,
- on clive à nouveau le groupe protecteur introduit au deuxième stade, d'une manière usuelle (par exemple par agitation avec de l'acide acétique),
- on acétalise la fonction cétone d'une manière usuelle (par exemple par réaction avec le 2-méthyl-2-propyl-1,3-propane diol),
- on convertit en mésylate la fonction OH libre par réaction avec le chlorure de méthanesulfonyle dans la pyridine,
- on soumet le produit intermédiaire ainsi obtenu à un arrangement-1,2-réductif en présence d'aluminohydrure de disobutyle et de triéthylaluminium tout en conservant la stéréochromie, et
- on transforme le composé déhydro ainsi obtenu par hydrogénation catalytique, en 5-méthyl-2-(1-méthylbutyl) -5-propyl-1,3-dioxane optiquement actif.